(19) Europäisches Patentamt
European Patent Office
Office européen des brevets

(11) Veröffentlichungsnummer : **0 076 448**
**B1**

(12) **EUROPÄISCHE PATENTSCHRIFT**

(45) Veröffentlichungstag der Patentschrift : **19.12.84**

(21) Anmeldenummer : **82108913.3**

(22) Anmeldetag : **27.09.82**

(51) Int. Cl.³ : **C 07 C 19/02**, C 07 C 53/19, C 07 D311/72

# (54) Optisch aktive Bausteine für die Synthese der Seitenkette von (R,R,R)-Alpha-Tocopherol sowie Verfahren zu deren Herstellung.

(30) Priorität : **02.10.81 DE 3139238**

(43) Veröffentlichungstag der Anmeldung : **13.04.83 Patentblatt 83/15**

(45) Bekanntmachung des Hinweises auf die Patenterteilung : **19.12.84 Patentblatt 84/51**

(84) Benannte Vertragsstaaten : **CH DE FR GB LI NL**

(56) Entgegenhaltungen :
**GB-A- 1 456 830**
**US-A- 4 191 842**
**Chemical Abstracts Band 80, Nr. 11, 18. März 1974 Columbus, Ohio, USA Seite 320, Spalte 2, Abstract Nr. 59476q**
**CHEMISCHE BERICHTE, Band 91, Nr. 10, 1958, Weinheim-Bergstrasse F. ASINGER et al. "Zum Problem der Sulfochlorierung am tertiären Kohlenstoffatom" Seiten 2130 bis 2136**
**Chemical Abstracts Band 89, Nr. 15, 9. Oktober 1978 Columbus, Ohio, USA C.A. HENRICK et al. "Ethyl (E)-3,5-ethano-7,11-dimethyl-2,4-dodecadienoate, a new insect growth regulator with potent juvenile hormone activity" Seite 177, Spalte 2, Abstract Nr. 124523e**
**Houben-Weyl "Methoden der organischen Chemie" Bd. 4/2 (1955) Seite 530, Zeilen 11-16**

(73) Patentinhaber : **BASF Aktiengesellschaft**
**Carl-Bosch-Strasse 38**
**D-6700 Ludwigshafen (DE)**

(72) Erfinder : **Ernst, Hansgeorg, Dr.**
**Bruesseler Ring 38**
**D-6700 Ludwigshafen (DE)**
Erfinder : **Vogel, Friedrich, Dr.**
**Im kleinen Letten 3**
**D-6706 Wachenheim (DE)**
Erfinder : **Paust, Joachim, Dr.**
**Ringstrasse 3**
**D-6708 Neuhofen (DE)**

**EP 0 076 448 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft (R)-(+)-β-Chlor-isobuttersäure (II), (S)-(+)-1-Brom-3-chlor-2-methyl-propan (IV) und (2R)-(+)-1-Chlor-2,6-dimethyl-heptan (V) sowie ein Verfahren zur Herstellung von (2R,6R)-1-Chlor-2,6,10-trimethyl)-undecan (I) ausgehend von II über die neuen Verbindungen IV und V und die Verwendung der neuen Verbindungen II, IV und V als optisch aktive Bausteine für die Synthese der Seitenkette von natürlichem optisch aktivem Vitamin E, dem (2R,4'R,8'R)-α-Tocopherol der Formel

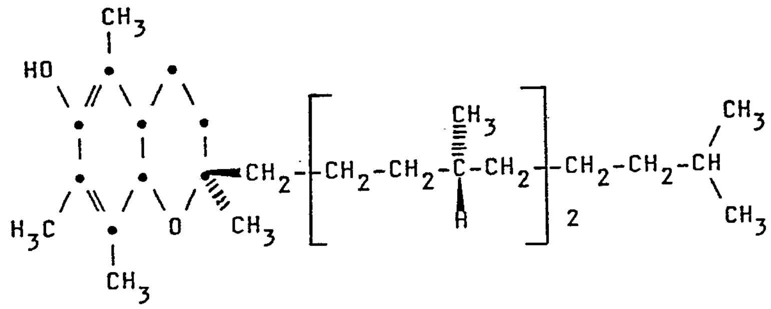

Die Substituenten der oben angegebenen optisch aktiven Strukturformel sowie der übrigen in dieser Anmeldung benutzten optisch aktiven Strukturformeln sind, sofern sie vor der Ebene des Moleküls liegen, durch das Zeichen ▲, sofern sie hinter der Ebene des Moleküls liegen, durch das Zeichen ≡ gekennzeichnet. Die Substituenten der stereochemisch nicht besonders gekennzeichneten Strukturformeln können entweder R oder S orientiert sein, oder die Verbindung kann als Gemisch der R- und S-Isomeren vorliegen.

Die Verbindungen II, IV und V waren in Form ihrer Racemate bereits bekannt. So wird die β-Chlor-isobuttersäure in Chem. Abstracts 80 (1974), Seite 320, Abstract Nr. 59 476 q ; das 3-Chlor-1-brom-2-methyl-propan in Chem. Berichte *91* (1958), Seite 2136 und das 1-Chlor-2,6-dimethyl-heptan in Bioorg. Chem. *7* (1978), Seite 245 beschrieben. Jedoch ist das Vorliegen racemischer Verbindungen im Hinblick auf die Aufgabe der vorliegenden Erfindung, einen technisch weniger aufwendigen Syntheseweg zu einem optisch aktiven $C_{14}$-Seitenkettenzwischenprodukt, das mit einem geeigneten Chroman-2-carboxaldehyd zu (2R,4'R,6'R)-α-Tocopherol umgesetzt werden kann, ohne Bedeutung, zumal wenn es sich um Verbindungen handelt, die zu einer Stoffgruppe gehören, von der man weiß, daß deren Aufspaltung in optisch aktive Isomere allgemein sehr problematisch ist (vgl. E. L. Eliel in « Stereochemie der Kohlenstoffverbindungen » (1969), Seite 69, erster Abschnitt und Houben-Weyl, « Methoden der organischen Chemie », 4/2 (1955), Seite 530, Zeilen 11 bis 16).

Aus J. Org. Chem. Vol. *41* No. 22 (1976), Seite 3505 f bzw. DE-OS 26 02 507 ist es bekannt, daß man natürliches Vitamin E durch Umsetzen eines Chroman-2-carboxaldehydes der Formel VIII

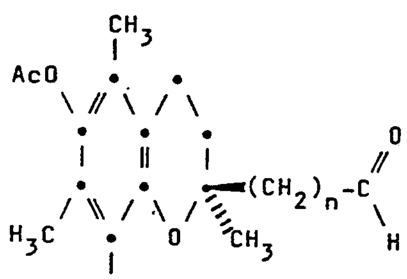

(VIIIa : n = 0)
(VIIIb : n = 1)

mit dem entsprechenden optisch aktiven $C_{15}$- bzw. $C_{14}$-Seitenkettenzwischenprodukt mittels einer Wittig-Reaktion herstellen kann. Die Seitenkette von natürlichem Vitamin E besitzt 2 Asymmetriezentren, die beide R-Konfiguration aufweisen. Zur Herstellung eines optisch aktiven Seitenkettenzwischenprodukts mit 14 C-Atomen sind Verfahren bekannt, die von optisch aktiven Bausteinen geringerer Kohlenstoffzahl ausgehen und durch zweifache Verwendung eines solchen chiralen Bausteines die beiden Asymmetriezentren herstellen (vgl. loc. cit.). Diese Verfahren gehen im Prinzip von der (S)-(+)-3-Hydroxy-2-methyl-propansäure aus, die auf die in Schema 1 skizzierte Weise über eine Veretherung/Veresterung mit Bortrifluorid/Isobutylen zu X, Alanatreduktion zu XI und Bromierung in den optisch aktiven $C_4$-Baustein (S)-(+)-3-tert.-Butoxy-2-methyl-1-brom-propan XIII überführt wird. XIII wird um eine $C_1$-Einheit verlängert, durch Umsetzen mit NaCN zum Nitril XIV und anschließende Reduktion des Nitrils zum Aldehyd XV bzw. durch Nitrilverseifung zur Säure XX, deren Reduktion zum Carbinol XXI und anschließende Tosylierung zu dem Tosylat XXII. Aus dem Aldehyd XV wird durch Wittig-Reaktion mit Isobutyltriphenylphosphoniumjodid,

Schema 1

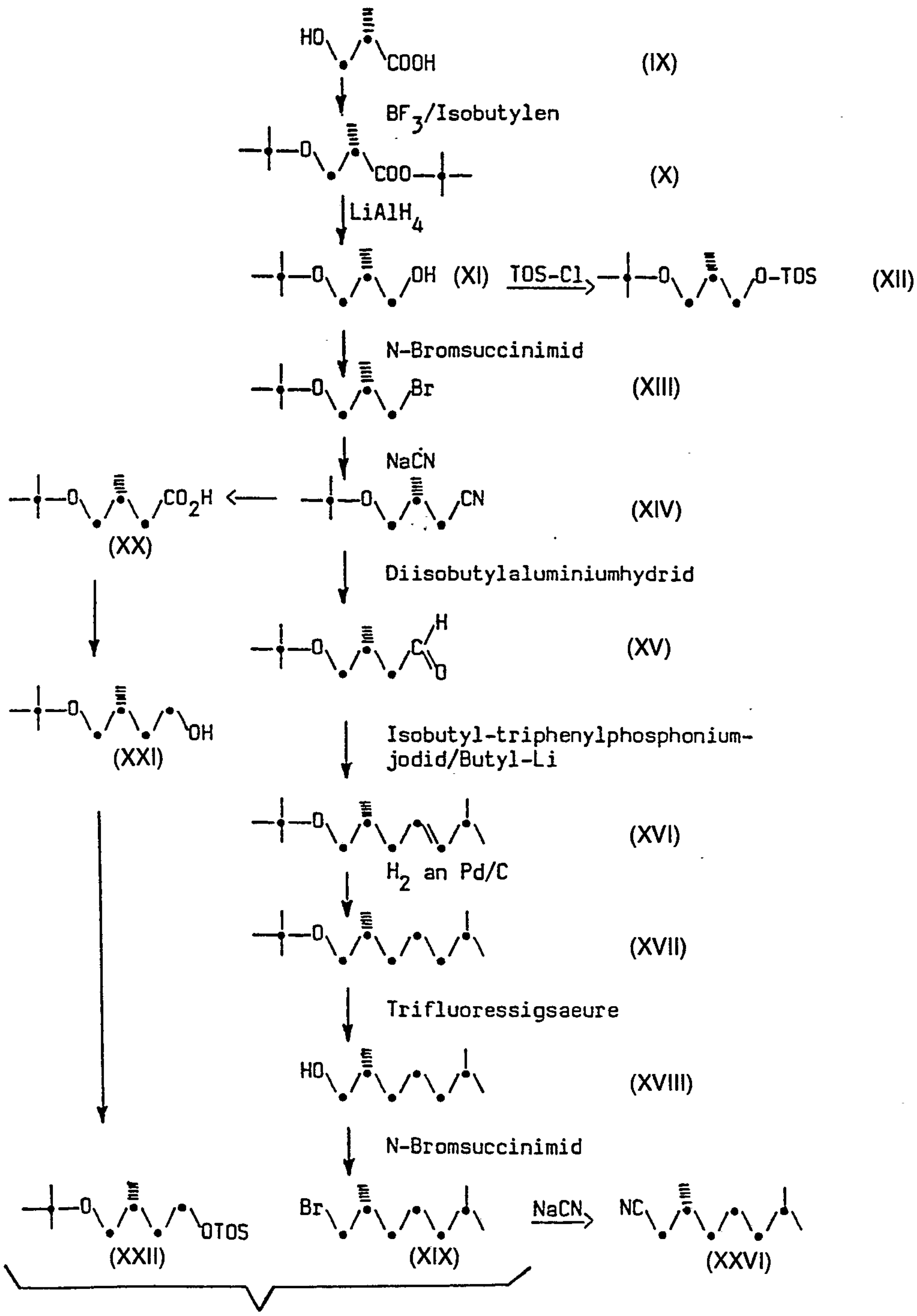

HO
COOH
(IX)
BF3/Isobutylen
COO
(X)
LiAlH4
OH
(XI)
TOS-Cl
O-TOS
(XII)
N-Bromsuccinimid
Br
(XIII)
NaCN
CO2H
(XX)
CN
(XIV)
Diisobutylaluminiumhydrid
H
(XV)
OH
(XXI)
Isobutyl-triphenylphosphonium-
jodid/Butyl-Li
(XVI)
H2 an Pd/C
(XVII)
Trifluoressigsaeure
HO
(XVIII)
N-Bromsuccinimid
OTOS
(XXII)
Br
(XIX)
NaCN
NC
(XXVI)

Schema 1 (Fortsetzung)

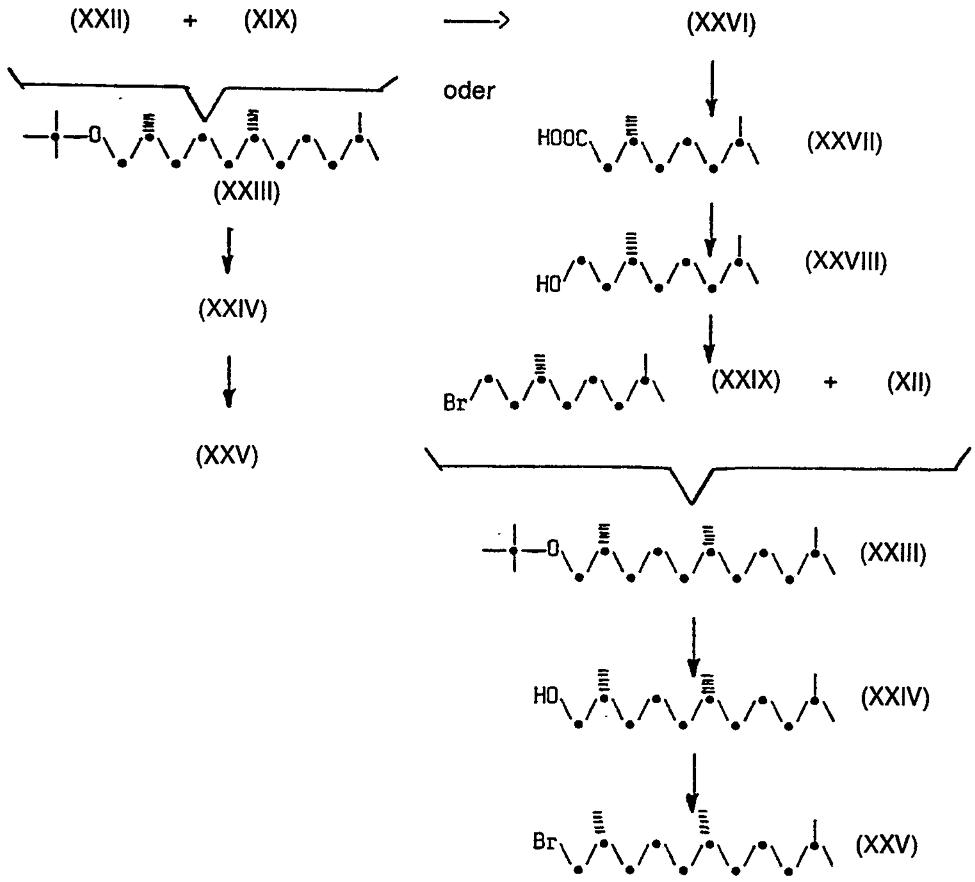

(XXII) + (XIX) → (XXVI)

oder

(XXIII)

(XXIV)

(XXV)

(XXVII)

(XXVIII)

(XXIX) + (XII)

(XXIII)

(XXIV)

(XXV)

(TOS = 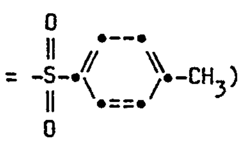)

Hydrierung, Butyletherspaltung und Bromierung das $C_9$-Bromid XIX hergestellt, das mit dem Tosylat XXII zu dem $C_{14}$-Ether gekuppelt wird, welcher nach Etherspaltung zu dem $C_{14}$-Bromid XXV bromiert werden kann.

Alternativ wird die $C_1$-Einheit mit Cyanid auf der Stufe des $C_9$-Bromids XIX eingeführt. Das Nitril XXVI wird durch Verseifung, Reduktion und Bromierung in das $C_{10}$-Bromid XXIX überführt, welches dann mit dem aus XI hergestellten Tosylat XII zu dem $C_{14}$-Ether XXIII verbunden werden kann.

Nachteilig an diesem Verfahren ist, einerseits, daß die als Ausgangsverbindung benötigte (S)-(+)-3-Hydroxy-2-methylpropansäure bisher nur durch bakterielle Oxidation von Isobuttersäure hergestellt werden kann und solche mikrobiologischen Verfahren technisch recht aufwendig und teuer sind, oder aber durch Racematspaltung in nur sehr schlechter Ausbeute (vgl. Biochem. Zeitschr. *342* (1965), S. 256 und 265) gewonnen werden kann, und andererseits, daß zur Herstellung des optisch aktiven $C_{14}$-Bromids XXV, je nach Variante, 15 bis 17 Reaktionsstufen notwendig sind.

Weiterhin ist aus GB 1 456 830 ein Verfahren zur Herstellung von (2R,6R)-1-Halogen-2,6,10-trimethyl)-undecanen bekannt. Gemäß diesem Verfahren wird (2R,6R)-1-Brom-2,6,10-trimethyl)-undecan durch Bromierung des entsprechenden Alkohols hergestellt, welcher seinerseits durch Dehydratisieren, Ozonisieren und Reduzieren der erhaltenen Ozonide mit Metallhydridkomplexen von (7R,11R)-Phytol hergestellt wird. Nachteilig an diesem Verfahren ist, daß man von dem nur relativ schlecht zugänglichen optisch aktiven Phytol ausgehen muß.

Es war daher die Aufgabe der Erfindung einen technisch weniger aufwendigen Syntheseweg zu einem optisch aktiven $C_{14}$-Seitenkettenzwischenprodukt zu finden, das mit dem Chroman-2-carboxaldehyd der Formel VIII zu (2R,4'R,6R')-α-Tocopherol umgesetzt werden kann.

Mit der (2R)-(+)-β-Chlor-isobuttersäure II wurde eine relativ einfach zugängige optisch aktive Ausgangsverbindung gefunden, von der aus man in nur 6 Reaktionsstufen über die neuen optisch aktiven Zwischenprodukte (2S)-(+)-1-Brom-3-chlor-2-methyl-propan IV und (2R)-(+)-1-Chlor-2,6-dimethyl-heptan V zu dem optisch aktiven $C_{14}$-Baustein (2R,6R)-1-Chlor-2,6,10-trimethylundecan I gelangen kann.

Gegenstand der Erfindung ist neben den neuen optisch aktiven Zwischenprodukten II, IV und V und deren Verwendung als optisch aktive Bausteine für die Synthese der Seitenkette von (R,R,R)-α-Tocopherol ein Verfahren zur Herstellung des $C_{14}$-Bausteins (2R,6R)-1-Chlor-2,6,10-trimethyl-undecan der Formel I

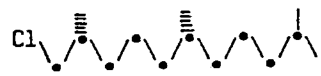
(I)

das dadurch gekennzeichnet ist, daß man

A) die neue (R)-(+)-β-Chlor-isobuttersäure der Formel II

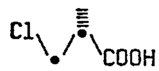
(II)

als optisch aktives Ausgangsmaterial verwendet,

B) dieses mit Borwasserstoff, Borwasserstoffderivaten oder Lithiumaluminiumhydrid, vorzugsweise mit Borwasserstoff oder Borwasserstoffderivaten, zum (R)-(−)-3-Chlor-2-methyl-propanol der Formel III

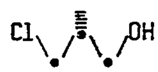
(III)

reduziert,

C) dieses mit Phosphortribromid, mit Triphenylphosphin und Brom in Dimethylformamid oder mit Triphenylphosphin und Tetrabromethan in Methylenchlorid zu dem neuen (S)-(+)-1-Brom-3-chlor-2-methyl-propan der Formel IV

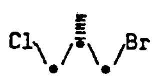
(IV)

bromiert,

D) dieses mit Isopentylmagnesiumbromid in einem inerten etherischen Lösungsmittel in Gegenwart von einem Dialkalimetall-tetrahalogenkuprat, vorzugsweise von Dilithiumtetrachlorokuprat in das neue (2R)-(+)-1-Chlor-2,6-dimethylheptan der Formel V

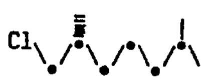
(V)

überführt,

E) dieses durch Behandeln mit metallischem Magnesium und nachfolgendes Einleiten von gasförmigem Formaldehyd in das (3R)-3,7-Dimethyl-octan-1-ol der Formel VI

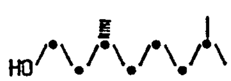
(VI)

überführt,

F) dieses analog C zu (3R)-1-Brom-3,7-dimethyloctan der Formel VII

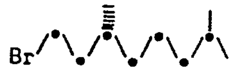
(VII)

bromiert und

G) dieses durch Behandeln mit metallischem Magnesium in die entsprechende Grignardverbindung überführt, welche in einem etherischen Lösungsmittel in Gegenwart von einem Dialkalimetall-tetrahalogenkuprat, vorzugsweise von Dilithiumtetrachlorokuprat mit (S)-(+)-1-Brom-3-chlor-2-methyl-propan zu I umgesetzt werden kann.

Das neue Verfahren zu dem $C_{14}$-Baustein I hat gegenüber den bekannten Verfahren zur Herstellung optisch aktiver $C_{14}$-Seitenkettenzwischenprodukte wesentliche Vorteile.

1. Man geht von technisch leichter zugänglichen optischaktivem Ausgangsmaterial aus.

2. Zur Durchführung der bekannten Verfahren sind ausgehend von dem optisch aktiven Ausgangs-

material IX bis zum $C_{14}$-Bromid XXV, je nach Variante, 15 bis 17 Reaktionsschritte notwendig, während man bei dem neuen Verfahren in nur 6 Reaktionsschritten von dem optischen aktiven Ausgangsmaterial II zu dem $C_{14}$-chlorid I gelangt.

3. Durch das Arbeiten mit Halogeniden als Zwischenprodukten entfällt die Notwendigkeit Hydroxylgruppen durch teure Schutzgruppe wie die tert.-Butyl-Gruppe oder die Tosylatgruppe zu schützen und die Ethergruppe anschließend in mehreren Stufen in ein Halogenid zu überführen.

4. Die Verwendung von Formaldehyd als $C_1$-Baustein bedeutet gegenüber der langen und aufwendigen Sequenz: Cyanidbildung — Verseifung — Reduktion — Halogenierung — eine erhebliche Verkürzung des Syntheseweges.

5. Im Hinblick auf die technische Realisierbarkeit des neuen Verfahrens ist besonders vorteilhaft, daß gleiche Reaktionsschritte wie Bromierungen und Grignard-Umsetzungen sich mehrfach wiederholen.

Das erfindungsgemäße Verfahren geht aus von (2R)-(+)-β-Chlor-isobuttersäure II als optisch aktivem Ausgangsmaterial. II kann aus der bereits bekannten und durch HCl-Addition an Methacrylsäure erhältlichen racemischen β-Chlor-isobuttersäure und Racematspaltung hergestellt werden (vgl. C.A. *68* (1968) 21 900 n).

Nach neuesten eigenen Ergebnissen gelingt die Herstellung der racemischen β-Chlor-isobuttersäure wesentlich vorteilhafter, wenn man — anstelle des gemäß locus citatus vorgesehenen Begasens einer etherischen Lösung von Methacrylsäure mit HCl bei 0 °C — die Methacrylsaüre im Autoklaven bei Temperaturen von 100 bis 160 °C mit konzentrierter wäßriger Salzsäure unter Eigendruck (bis etwa 15 bar) oder mit aufgepreßtem HCl-Gas (bis etwa 40 bar) umsetzt. Bei der Umsetzung mit wäßriger Salzsäure verwendet man im allgemeinen Lösungen von 10 bis 40 Gewichtsprozent Methacrylsäure in der üblichen konzentrierten Salzsäure. Bei dieser Verfahrensverbesserung verringert sich die Reaktionszeit bei etwa gleichen Ausbeuten von etwa 40 Stunden auf 1 bis 2 Stunden.

Die Racematspaltung gelingt vorteilhaft durch mehrmaliges Umkristallisieren des Gemisches der diastereomeren Salze der racemischen Säure mit d-Ephedrin aus organischen Lösungsmitteln wie Essigsäureethylester oder Toluol, anschließendes Spalten der Salze durch Auflösen in verdünnter Säure, wie HCl oder $H_2SO_4$, und Extraktion mit einem geeigneten organischen Lösungsmittel.

Das Gemisch der diastereomeren Salze erhält man durch Zugabe einer Lösung von d-Ephedrin in einem organischen Lösungsmittel, wie Diethylether, Diisopropylether, Methyl-tert.-butylether, Aceton, Methylethylketon, Toluol oder Essigsäureethylester zu einer Lösung der racemischen Säure in einem entsprechenden Lösungsmittel.

Das Prinzip, den zu verwendenden optisch aktiven $C_4$-Baustein über die Racematspaltung von β-Chlor-isobuttersäure zu gewinnen, ist nicht zuletzt deshalb vorteilhaft, da das nicht einsetzbare S-(−)-Isomere in einfacher Weise durch Erhitzen mit konzentrierter wäßriger Salzsäure oder HCl-Gas im Autoklaven auf 100 bis 160 °C racemisiert und so erneut in die Racematspaltung eingesetzt werden kann.

Die Reduktion der optisch aktiven Säure II zu dem Alkohol III kann mit Borwasserstoff oder Borwasserstoffderivaten, wie einem $BH_3$-Tetrahydrofuran-Komplex, einem $BH_3$-Dimethylsulfid-Komplex, gasförmigem Diboran, einer Kombination von $NaBH_4$ und $BF_3$ oder aber mit Lithiumaluminiumhydrid in Lösungsmitteln wie Diethylether, Tetrahydrofuran oder Diethylenglycoldimethylether (Diglyme) durchgeführt werden. Mit besonderem Vorteil gelingt die Reduktion mit Borwasserstoff oder Borwasserstoffderivaten, da diese auch unter milden Bedingungen sehr rasch verläuft, da sich die Aufarbeitung des Reaktionsansatzes wesentlich einfacher gestaltet (kein Ausfällen eines voluminösen Niederschlages) als die bei Verwendung von Lithiumaluminiumhydrid.

Die Hydride verwendet man im allgemeinen in Mengen von 0,3 bis 1,5 Mol pro Mol II. Die Reaktionstemperaturen betragen, im allgemeinen bis 50 °C, die Reaktionszeiten etwa 1 bis 6 Stunden.

Die Reduktion von β-Chlor-isobuttersäure zum 3-Chlor-2-methyl-propanol ist in der Literatur noch nicht beschrieben. Bekannt ist nur die Reduktion von β-Chlor-carbonsäuren wie 3-Chlor-3-methylbuttersäure und 3-Chlor-buttersäure mit Lithiumaluminiumhydrid (vgl. J. Am. Chem. Soc. *78* (1956) S. 4049), die allerdings nur mit unbefriedigenden Ausbeuten verläuft, da stets ein erheblicher Anteil der Säure während der Umsetzung dehalogeniert wird.

Bekannt sind weiterhin die Reduktion von 11-Brom-undecancarbonsäure mit einem Boran-Dimethylsulfid-Komplex zu 11-Brom-undecanol (vgl. Aldrichimica Acta *8* (1975) S. 20) und die Reduktion von rein aliphatischen Carbonsäuren oder α-Halogencarbonsäuren (Chloressigsäure) mit einen Boran-Tetrahydrofuran-Komplex (vgl. J. Org. Chem. *38*, No. 16 (1973) S. 2786 f).

Optisch aktives 3-Chlor-2-methyl-propanol wurde kürzlich in der FR-PS 79 10 589 (Publikationsnummer 2 455 017) und der FR-PS 79 10 050 (2 454 465) beschreiben, gemäß welcher es durch stereospezifische Hydrolyse eines Alkylesters mittels Pankreaslipase auf einem Trägermaterial hergestellt wird.

Die Bromierung des Alkohols III zu dem neuen (S)-(+)-1-Brom-3-chlor-2-methyl-propan IV gelingt mit Vorteil mit Phosphortribromid, mit einem Triphenylphosphin-Brom-Gemisch in Dimethylformamid oder mit einem Triphenylphosphin-Tetrabrommethan-Gemisch in Methylenchlorid. Sie kann aber auch mit HBr als Gas oder als konzentrierter wäßriger Lösung vorgenommen werden.

Die Bromierungsmittel verwendet man im allgemeinen in Mengen von 0,3 bis 2, vorzugsweise 0,5 bis 1,5 Mol pro Mol III. Die Reaktionstemperaturen liegen etwa zwischen 0 und 100, vorzugsweise 25 bis 90 °C, die Reaktionszeiten bei 1 bis 6 Stunden.

Die Umsetzung des Dihalogenids IV mit Isopentylmagnesiumbromid in einem inerten etherischen Lösungsmittel, in Gegenwart von katalytischen Mengen eines Dialkalimetall-tetrahalogenkuprats, führt überraschenderweise unter völlig selektiver Substitution des Broms zu dem Chlorid V. Als inertes etherisches Lösungsmittel verwendet man hierbei beispielsweise Dioxan, Diethylether, Diethylenglykoldimethylether oder Dimethoxyethan, vorzugsweise Tetrahydrofuran. Als Dialkalimetall-tetrahalogenkuprat verwendet man vorzugsweise Dilithiumtetrachlorokuprat. Das bei dieser Reaktion erhaltene 1-Chlor-2,6-dimethylheptan ist bisher auch nur als Racemat beschrieben (vgl. Bioorg. Chem. *7* (1978) S. 235 f., bes. 245).

Das Chlorid V wird in einem etherischen Lösungsmittel, vorzugsweise in Diethylether oder Tetrahydrofuran gelöst, und diese Lösung mit etwa äquimolekularen Mengen an metallischem Magnesium in einem etherischen Lösungsmittel behandelt. In das erhaltene Reaktionsgemisch wird nach ein bis mehrstündigem Erhitzen zum Sieden unter Rückfluß und Abkühlen gasförmiges Formaldehyd eingeleitet, das z. B. durch Erhitzen von trockenem Paraformaldehyd auf 180 °C erhalten werden kann, bis nach H. Gilman (J. Amer. Chem. Soc. *47,* 2002 (1925)) im Reaktionsgemisch kein Grignard-Reagens mehr nachgewiesen werden kann. Es werden 1 bis 1,5 Mol Formaldehyd aufgenommen, die Umsetzung dauert 30 bis 60 Minuten.

Das hierbei erhaltene (3R)-3,7-dimethyl-octan-1-ol VI kann auf übliche Weise aus dem Reaktionsgemisch isoliert werden. Beispielsweise wird das Reaktionsgemisch mit Eis und verdünnter Schwefelsäure versetzt und dann einer Wasserdampfdestillation unterworfen. Die wäßrige Phase des Destillats wird mit einem Ether extrahiert und die vereinigten organischen Phasen getrocknet und destilliert.

Die Bromierung des Alkohols VI zu dem (3R)-1-Brom-3,7-dimethyl-octan VII wird mit Vorteil nach einem der oben beschriebenen Bromierungsverfahren durchgeführt.

Das $C_{10}$-Bromid VII wird durch Behandeln mit metallischem Magnesium in einem etherischen Lösungsmittel in die entsprechende Grignard-Verbindung überführt und dann in Gegenwart von katalytischen Mengen eines Dialkalimetall-tetrahalogenkuprats, vorzugsweise des Dilithiumtetrachlorokuprats mit dem (S)-(+)-1-Brom-3-chlor-2-methyl-propan IV zu dem (2R,6R)-1-Chlor-2,6,10-trimethyl-undecan I gekuppelt.

Mit Hilfe des erfindungsgemäßen Verfahrens kann man ausgehend von dem neuen, gut zugänglichen optisch aktiven Ausgangsstoff (2R)-(+)-β-Chlor-isobuttersäure über neue optisch aktive Zwischenprodukte in nur 6 einfachen Reaktionsschritten (2R,6R)-1-Chlor-2,6,10-trimethyl-undecan I erhalten. I ist ein wichtiges Zwischenprodukt für die Synthese von (R,R,R)-α-Tocopherol.

Beispiel 1

Herstellung von (R)-(+)-β-Chlor-isobuttersäure

a) Herstellung racemischer β-Chlor-isobuttersäure

1,3 kg Methacrylsäure werden im Autoklaven mit 10 l konzentrierter wäßriger Salzsäure 1 Stunde bei 110 °C gerührt. Das Reaktionsgemisch wird anschließend abgekühlt, mit Methylenchlorid extrahiert, der Extrakt mit Wasser gewaschen, über Magnesiumsulfat getrocknet, eingeengt und schließlich bei 108 °C/20 mbar destilliert. Man erhält 1,69 kg β-Chlor-isobuttersäure, die nach gaschromatographischer Analyse (GC) 98 % rein ist. Das entspricht einer Ausbeute von 91 % der Theorie.

b) (R)-(+)-β-Chlor-isobuttersäure (II) aus racemischer β-Chlor-isobuttersäure

Zu 500 g (4,08 mol) (+)-β-Chlor-isobuttersäure in 400 ml Diisopropylether werden bei Raumtemperatur 300 g d-Ephedrin (1,82 mol) in 1 600 ml Diisopropylether gegeben. Es wird 1 Stunde unter Rückfluß zum Sieden erhitzt, 2 Stunden bei Raumtemperatur nachgerührt und bei 15 °C abgesaugt. Man erhält 478 g (92 %) d-Ephedrin-Salz.

Das Salz wird zehnmal aus Essigester umkristallisiert, bis der Drehwert konstant bleibt. Man erhält 35,0 g d-Ephedrin-Salz vom Fp. = 124 bis 126 °C.

$$[\alpha]_D^{25} = +\ 30{,}47° \ (C = 5{,}106/CH_3OH)$$

Das Ephedrin-Salz wird in 2 n-Salzsäure gelöst ; es wird mit Ether extrahiert, mit 2 n-Salzsäure gewaschen, über Natriumsulfat getrocknet, eingeengt und anschließend destilliert. Man erhält 13,1 g II vom Siedepunkt Kp = 107 °C bei 16 mbar.

$$[\alpha]_D^{25} = 12{,}73° \ (C = 5{,}854/CH_3OH)$$

Beispiel 2

(R)-(−)-3-Chloro-2-methyl-propanol (III)

Zu einer Suspension von 3,8 g (0,1 mol) Natriumborhydrid in 100 ml Tetrahydrofuran werden bei

Raumtemperatur innerhalb 1 Stunde 12,3 g (0,1 mol) (R)-(+)-β-Chlor-isobuttersäure getropft. Es wird 30 Minuten bei Raumtemperatur nachgerührt. Dann wird eine Lösung aus 17 g (0,12 mol) Bortrifluorid-Etherat in 20 ml Tetrahydrofuran in 1 Stunde zugetropft, wobei das Reaktionsgemisch auf Raumtemperatur gehalten wird. Es wird 2 Stunden bei Raumtemperatur nachgerührt. Dann wird auf Eis gegossen, ausgeethert, mit Natriumhydrogencarbonat säurefrei gewaschen, getrocknet und eingeengt.

Der Rückstand wird destillativ gereinigt. Man erhält 7,8 g III vom Siedepunkt 77 °C bei 28 mbar, entsprechend einer Ausbeute von 72 % d. Th.

$$[\alpha]_D^{25} = -13{,}0° \quad (C = 4{,}82/CH_3OH)$$

Beispiel 3

(S)-(+)-1-Brom-3-chlor-2-methyl-propan (IV)

46,7 g (R)-(+)-3-Chlor-2-methyl-propanol (nach GC 97 %ig = 0,418 mol) werden bei 0 bis 10 °C innerhalb von 30 Minuten mit 54,2 g (0,2 mol) Phosphortribromid versetzt. Anschließend wird das Reaktionsgemisch 5 Stunden bei 90 °C gerührt, dann auf Eis gegossen, mit Petrolether extrahiert, mit Bicarbonatlösung gewaschen, über Natriumsulfat getrocknet und eingeengt.

Der Rückstand wird destillativ gereinigt. Man erhält 53,4 g vom Siedepunkt Kp = 53 °C bei 24 mbar, entsprechend einer Ausbeute von 74 % d. Th.

$$[\alpha]_D^{25} = 0{,}36° \quad (C = 5{,}043/CH_3OH)$$

Beispiel 4

(2R)-(+)-1-Chloro-2,6-dimethylheptan V

Zu 22 g (0,905 mol) Magnesium in 100 ml Tetrahydrofuran werden 135 g (0,89 mol) 1-Brom-3-methylbutan in 300 ml Tetrahydrofuran getropft. das Gemisch wird 1 1/2 Stunden bei 40 °C nachgerührt. Dann werden bei − 15 °C 63,6 g (0,37 mol) (S)-(+)-1-Brom-3-chlor-2-methyl-propan in 50 ml Tetrahydrofuran und anschließend 34,8 ml einer 0,5 molaren Lösung von Dilithiumtetrachlorokuprat in Tetrahydrofuran zugetropft. Es wird 3 Stunden bei − 15 °C nachgerührt und über Nacht bei Raumtemperatur stehengelassen. Es wird mit ca. 30 ml 30 %iger Schwefelsäure angesäuert und das Gemisch einer Wasserdampfdestillation unterworfen. Die organische Phase im Destillat wird abgetrennt, die wäßrige Phase mit Ether extrahiert. Die vereinigten organischen Phasen werden über Natriumsulfat getrocknet und eingeengt. Man erhält 74,5 g Rückstand, der nach GC 65 % V enthält, entsprechend einer Ausbeute von 80,5 % der Theorie.

Der Rückstand wird destillativ gereinigt. Man erhält 44,4 g vom Siedepunkt Kp = 72 °C bei 16 mbar (74 % d. Th.).

$$[\alpha]_D^{25} = +2{,}15 \quad (C = 2{,}925/CH_3OH)$$

Beispiel 5

(3R)-3,7-Dimethyloctan-1-ol (VI)

Zu 2 g Magnesium in 10 ml Tetrahydrofuran werden 9,92 g (2R)-(+)-1-Chloro-2,6-dimethylheptan in 35 ml Tetrahydrofuran zugegeben. Das Gemisch wird 2 1/2 Stunden unter Rückfluß zum Sieden erhitzt. Anschließend wird bei Raumtemperatur gasförmiges Formaldehyd eingeleitet, das durch Erhitzen von 3,5 g trockenem Paraformaldehyd auf 180 °C erzeugt wird.

Das Reaktionsgemisch wird mit 10 g Eis und 6 ml 30 %iger Schwefelsäure versetzt und einer Wasserdampfdestillation unterworfen. Die wäßrige Phase des Destillats wird zweimal mit Ether extrahiert; die vereinigten organischen Phasen werden über Natriumsulfat getrocknet und eingeengt. Man erhält 8,56 g Rückstand, der nach GC 91 % VI enthalt, entsprechend einer Ausbeute von 81 % der Theorie.

Der Rückstand wird durch Destillation gereinigt. Man erhält 6,7 g (= 70 % d. Th.) vom Siedepunkt 80 °C bei 6 mbar

$$[\alpha]_D^{25} = 4{,}9° \quad (pur)$$

Beispiel 6

(3R)-1-Brom-3,7-dimethyloctan (VII)

Zu 7,6 g (0,048 mol) (R)-3,7-Dimethyloctan-1-ol werden bei 5 bis 10 °C 5,77 g (0,022 mol) Phosphortribromid getropft. Dann wird 6 Stunden bei 100 °C gerührt. Man läßt abkühlen, gießt auf Eis,

extrahiert mit Petrolether, wäscht mit Bicarbonatlösung, trocknet über Natriumsulfat und engt ein. Man erhält 11,3 g Rückstand der nach GC 81,4 % Bromid VII enthält, entsprechend einer Ausbeute von 87 % der Theorie.

Der Rückstand wird durch Destillation gereinigt. Man erhält 7,9 g vom Siedepunkt 84 °C bei 8 mbar (75 % d. Th.)

$$[\alpha]_D^{25} = -5{,}93° \text{ (pur)}$$

Beispiel 7

(2R,6R)-1-Chlor-2,6,10-trimethyl-undecan (I)

Zu 2,0 g (0,082 mol) Magnesium in 50 ml Tetrahydrofuran werden 16,4 g (0,074 mol) (3R)-1-Brom-3,7-dimethyloctan getropft. Es wird 1 1/2 Stunden bei 40 °C nachgerührt und dann auf – 15 °C abgekühlt. Bei dieser Temperatur werden 7,42 g (0,043 mol) (S)-(+)-1-Brom-3-chlor-2-methyl-propan und anschließend 2,1 ml einer 0,5-molaren Lösung von Dilithiumtetrachlorokuprat in Tetrahydrofuran zugegeben. Es wird 2 Stunden bei – 15 °C nachgerührt ; dann läßt man auf Raumtemperatur kommen. Das Reaktionsgemisch wird mit 15 ml gesättigter Ammoniumchloridlösung versetzt, mit Ether extrahiert, über Natriumsulfat getrocknet und eingeengt.

Man erhält 7,4 g vom Siedepunkt Kp = 72 bis 75 °C bei 0,1 mbar, entsprechend einer Ausbeute von 74 % der Theorie.

$$[\alpha]_D^{25} = +2{,}2° \text{ (C = 1,890/}CHCl_3\text{)}$$

**Ansprüche**

1. Verfahren zur Herstellung von (2R,6R)-1-Chlor-2,6,10-trimethyl-undecan der Formel

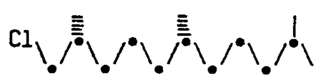

(I)

dadurch gekennzeichnet, daß man

A) die neue (R)-(+)-β-Chlor-isobuttersäure der Formel II

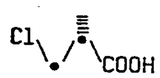

(II)

als optisch aktives Ausgangsmaterial verwendet,

B) dieses mit Borwasserstoff, Borwasserstoffderivaten oder Lithiumaluminiumhydrid zum (R)-(–)-3-Chlor-2-methyl-propanol der Formel III

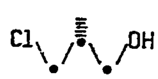

(III)

reduziert,

C) dieses mit Phosphortribromid, mit Triphenylphosphin und Brom in Dimethylformamid oder mit Triphenylphosphin und Tretrabrommethan in Methylenchlorid zu dem neuen (S)-(+)-1-Brom-3-chlor-2-methyl-propan der Formel IV

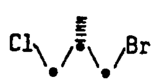

(IV)

bromiert,

D) dieses mit Isopentylmagnesiumbromid in einem inerten etherischen Lösungsmittel in Gegenwart von einem Dialkalimetall-tetrahalogenkuprat, vorzugsweise von Dilithiumtetrachlorokuprat in das neue (2R)-(+)-1-Chlor-2,6-dimethylheptan der Formel V

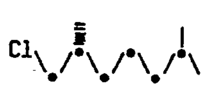

(V)

überführt,

E) dieses durch Behandeln mit metallischen Magnesium in etherischer Lösung und nachfolgendes Einleiten von gasförmigen Formaldehyd in das (3R)-3,7-Dimethyl-octan-1-ol der Formel VI überführt,

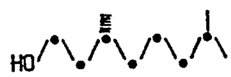 (VI)

F) dieses analog C zu (3R)-1-Brom-3,7-dimethyl-octan der Formel VII

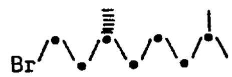 (VII)

bromiert und

G) dieses durch Behandeln mit metallischem Magnesium in die entsprechende Grignard-Verbindung überführt, welche in einem etherischen Lösungsmittel in Gegenwart von einem Dialkalimetalltetrahalogenkuprat, vorzugsweise von Dilithiumtetrachlorokuprat mit (S)-(+)-1-Brom-3-chlor-2-methyl-propan zu I umgesetzt werden kann.

2. (R)-(+)-β-Chlor-isobuttersäure (II).

3. (S)-(+)-1-Brom-3-chlor-2-methyl-propan (IV).

4. (2R)-(+)-1-Chlor-2,6-dimethyl-heptan (V).

5. Verwendung der Verbindungen gemäß den Ansprüchen 2 bis 4 als optisch aktive Bausteine für die Synthese der Seitenketten von (R,R,R)-α-Tocopherol.

**Claims**

1. A process for the preparation of (2R,6R)-1-chloro-2,6,10-trimethyl-undecane of the formula

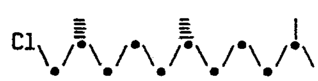 (I)

wherein

A) the novel (R)-(+)-β-chloro-isobutyric acid of the formula II

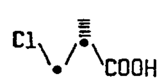 (II)

is used as the optically active starting material,

B) this starting material is reduced with boron hydride, boron hydride derivatives or lithium aluminium hydride, to give (R)-(−)-3-chloro-2-methyl-propanol of the formula III

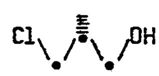 (III)

C) this compound is brominated with phosphorus tribromide, with triphenylphosphine and bromine is dimethylformamide, or with triphenylphosphine and carbon tetrabromide in methylene chloride to give the novel (S)-(+)-1-bromo-3-chloro-2-methyl-propane of the formula IV

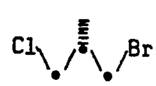 (IV)

D) this compound is converted into the novel (2R)-(+)-1-chloro-2,6-dimethylheptane of the formula V

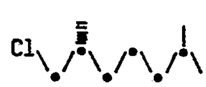 (V)

with isopentyl-magnesium bromide in an inert ethereal solvent in the presence of a di-alkali metal tetrahalocuprate, preferably dilithium tetrachlorocuprate,

E) the product in D is converted into (3R)-3,7-dimethyl-octan-1-ol of the formula VI

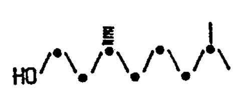 (VI)

by treatment with metallic magnesium in ethereal solution and subsequent introduction of gaseous formaldehyde,

F) the product in E is brominated by a method similar to that in C to give (3R)-1-bromo-3,7-dimethyloctane of the formula VII

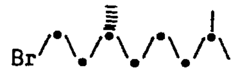

(VII)

and

G) this compound is converted, by treatment with metallic magnesium, into the corresponding Grignard compound, which can be reacted with (S)-(+)-1-bromo-3-chloro-2-methylpropane in an ethereal solvent in the presence of a dialkali metal tetrahalocuprate, preferably dilithium tetrachlorocuprate, to give I.

2. (R)-(+)-β-Chloro-isobutyric acid (II).

3. (S)-(+)-1-Bromo-3-chloro-2-methylpropane (IV).

4. (2R)-(+)-1-Chloro-2,6-dimethyl-heptane (V).

5. The use of compound as claimed in claims 2 to 4 as an optically active unit for the synthesis of the side chains of (R,R,R)-α-tocopherol.

**Revendications**

1. Procédé de préparation de (2R,6R)-1-chloro-2,6,10-triméthyl-undécane répondant à la formule

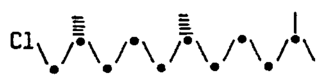

(I)

caractérisé en ce que

A) on utilise le nouvel acide (R)-(+)-β-chloro-isobutyrique répondant à la formule II

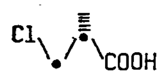

(II)

comme matière de départ optiquement active,

B) on la réduit avec de l'hydrogène boré, des dérivés de l'hydrogène boré ou de l'hydrure d'aluminium-lithium en (R)-(−)-3-chloro-2-méthyl-propanol répondant à la formule III

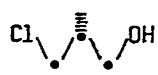

(III)

C) on brome ce dernier avec du tribromure de phosphore, de la triphénylphosphine et du brome dans du diméthylformamide, ou de la triphénylphosphine et du tétrabromométhane dans du chlorure de méthylène, obtenant ainsi le nouveau (S)-(+)-1bromo-3-chloro-2-méthylpropane répondant à la formule IV

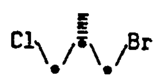

(IV)

D) on transforme celui-ci, avec du bromure d'isopentyl-magnésium dans un solvant éthérique inerte, en présence d'un tétrahalocuprate dialcalin, de préférence du tétrachlorocuprate dilithique, en le nouveau (2R)-(+)-1-chloro-2,6-diméthylheptane répondant à la formule V

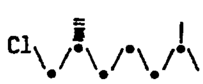

(V)

E) on transforme ce dernier, par traitement par du magnésium métallique en solution éthérique, puis introduction de formaldéhyde gazeux, en le (3R)-3,7-diméthyl-octane-1-ol répondant à la formule VI

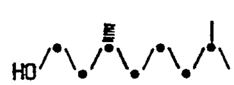

(VI)

F) on brome ce dernier, de façon analogue à C, en (3R)-1-bromo-3, 7-diméthyl-octane répondant à la formule VII

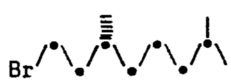

(VII)

et

G) on transforme ce dernier, par traitement par du magnésium métallique, en le composé organomagnésien correspondant, que l'on peut faire réagir, dans un solvant éthérique, en présence d'un tétrahalocuprate dialcalin, de préférence le tétrachlorocuprate dilithique, sur le (S)-(+)-1-bromo-3-chloro-2-méthyl-propane, pour obtenir I.

2. Acide (R)-(+)-β-chloro-isobutyrique (II).

3. (S)-(+)-1-bromo-3-chloro-2-méthyl-propane (IV).

4. (2R)-(+)-1-chloro-2,6-diméthyl-heptane (V).

5. Utilisation des composés selon les revendications 2 à 4 comme composants optiquement actifs pour la synthèse des chaînes latérales de (R,R,R)-α-tocophérol.